# EUROPEAN PATENT APPLICATION

(11) **EP 1 374 876 A1**
(43) Date of publication of application: **02.01.2004**
(21) Application number: 02701720.1
(22) Date of filing: 05.03.2002
(51) Int. Cl.: A61K 35/14, A61K 41/00, A61P 35/00, A61P 43/00

(54) **METHOD OF TREATING TUMOR AND CONSIGNMENT SYSTEM OF PROLIFERATING AND PROCESSING ACTIVATED LYMPHOCYTES TO BE USED IN PARALLEL TO PDT**

(30) Priority: 05.03.2001 JP 2001059408
(71) Applicant: Lymphotec Inc., Tokyo 112-0001 (JP)
(72) Inventor: YANAI, Hideo, Ube-shi, Yamaguchi 755-0047 (JP); OKITA, Kiwamu, Ube-shi, Yamaguchi 755-0151 (JP); KUROIWA, Yasuyuki, Hitachinaka-shi, Ibaraki 312-0062 (JP); SEKINE, Teruaki, Koto-ku, Tokyo 135-0043 (JP)
(74) Representative: Eddowes, Simon
(86) International application number: PCT/JP2002/001997
(87) International publication number: WO 2002/069990

(57) **Abstract**

A treatment method more effective in treating tumors such as cancer by administering activated lymphocytes in combination with a photodynamic therapy procedure within a specific period of time preceding or following the procedure is provided. By treating the patient with the combination of PDT procedure and the activated lymphocyte administration, a remarkable effect is achieved in reducing and suppressing various types of tumors, including stomach cancer in particular, and also desirable results are achieved even when tumors not responsive to PDT procedure alone are treated in this manner, due to the synergy of the PDT procedure and the activated lymphocyte administration. Provided that the activated lymphocytes are used in combination with a PDT procedure, even activated lymphocytes not necessarily achieving cancer specificity can be expected to improve the treatment effect in destroying or suppressing the growth of tumors such as cancers.

## Description

### TECHNICAL FIELD

The present invention relates to a therapy method that may be adopted to greatly improve the effect of treatment for tumors such as cancer through a photodynamic therapy (PDT) and a system for propagating / processing lymphocytes on assignment to obtain activated lymphocytes for use in conjunction with PDT treatment. More specifically, it relates to a therapy method that achieves a remarkable improvement in the effect of the treatment of a tumor such as cancer by administering activated lymphocytes over a specific period of time before or after the implementation of PDT.

### BACKGROUND ART

The latest methods for treating tumors such as cancer that have become standard options in addition to the conventional treatments such as surgical excision and carcinostatic administration include administration of lymphocytes and laser irradiation treatments. The inventor of the present invention has already reported with regard to the lymphocyte administration (see Japanese Unexamined Patent Publication No. H 3-80076) that lymphocytes originating from peripheral blood or the like can be propagated by using fixed anti-CD 3 antibodies or interleukin 2 and that the lymphocytes thus propagated have an anti-neoplastic effect.

The laser irradiation therapy method, through which cancer is necrotized by administering a carcinophilic photosensitive drug in advance and then radiating laser light on the cancerous area, thus generating active oxygen in the cancerous tissue through photodynamic therapy (hereafter, it may be simply referred to as "PDT") has been attracting a great deal of interest since it has the advantage of being less invasive and enables a cancer treatment for patients who are not suitable for surgical intervention.

Although the data obtained through the research to date demonstrate somewhat inconsistent results due to the small number of available cases that have been studied, it has been reported that cure rates of 50% and 89% were respectively achieved for prophase patients and anaphase patients with stomach cancer at early stages through PDT, while data of the two prophase cases of progressive stomach cancer were all deemed invalid and four cases out of the 5 anaphase progressive stomach cancer cases studied were deemed invalid (Cancer and Chemotherapy (1996): Volume 23, No. 1, pp 41 ∼ 46).

In addition, it has been reported that while a transfusion of mouse spleen unimmunized with cancerous cells did not inhibit post-PDT growth of cancerous cells transplanted into SCID (severe combined immunodeficient) mice, post-PDT growth of cancerous cells transplanted in SCID mice could be inhibited by transfusing the spleen of mice immunized with cancerous cells (Cancer Research (1999), 59, 1941-1946). It is generally known that the results of animal testing conducted by using mice are often very different from the results of treatment on humans.

However, the therapy mentioned earlier, in which lymphocytes having been simply activated are used as an anti-neoplastic activator, does not achieve a very good anti-neoplastic effect and, furthermore, does not contribute to the destruction of the tumor or the prevention of tumor growth in the PDT treatment. While lymphocytes may conceivably be prepared by inducing cancer specificity so as to react specifically to cancer, there are problems such as 1) the preparation process is complicated, 2) the type of cancerous cells required for such induction is not always available and 3) lymphocytes achieving such specificity cannot always be prepared with consistent reliability through a given preparation procedure, and for these reasons, the concept has not yet been put into practical application. Moreover, the PDT treatment itself is not always effective in treating the target cancer, as indicated by the research data discussed earlier.

### DISCLOSURE OF THE INVENTION

Accordingly, the present invention provides a therapy method in which a tumor is treated by administering activated lymphocytes in conjunction with PDT (photodynamic therapy). By administering activated lymphocytes simultaneously during PDT or over a specific period of time before or after the PDT, a tumor such as cancer can be treated with the activated lymphocytes that may not necessarily have cancer specificity to destroy the tumor or to greatly inhibit the growth of the tumor.

It is desirable to treat the patient with PDT repeatedly over a plurality of sessions, and a marked tumor treatment effect can be expected when the activated lymphocytes are administered by in conjunction with PDT treatment within 6 months following or prior to the PDT treatment.

The activated lymphocytes to be used in conjunction with PDT may be propagated or activated in a culture by using anti-CD 3 antibodies or interleukin 2.

A system for propagating / processing lymphocytes to obtain activated lymphocytes for use in conjunction with PDT, in which cells contained in sampled blood, bodily fluid or tissue provided by a client unit requesting activated lymphocytes are propagated or activated at a processing unit to prepare the activated lymphocytes to be used in conjunction with PDT and the activated lymphocytes are saved or supplied to the client unit, can be achieved. Such a system of assigned propagation processing constitutes a rational system through which the processing unit such as a preparation processing contractor delivers activated lymphocytes to the client in response to an order issued by the client, who may be a medical institute or the like needing the activated lymphocytes.

### BEST MODE FOR CARRYING OUT THE INVENTION

The following is an explanation of specific details of the present invention, given in reference to an embodiment thereof. The present invention was conceived and completed by the inventor who, for the first time, discovered that a preparation of lymphocytes activated by using anti-CD 3 antibodies and interleukin 2 in advance achieved an outstanding anti-neoplastic affect in treating cancer that did not respond to an initial PDT when the administration of the activated lymphocytes was combined with PDT even though the lymphocytes were not cancer specific lymphocytes.

In more specific terms, activated lymphocytes not necessarily achieving cancer specificity, which are prepared by propagating and activating lymphocytes with anti-CD 3 antibodies or interleukin 2 to be administered in combination with PDT are administered over a specific period before or after the PDT procedure to effectively treat a tumor such as cancer through the synergy of PDT and the activated lymphocyte administration implemented in combination.

### (Harvesting Lymphocytes)

The lymphocytes used in this treatment do not need to achieve cancer specificity and thus, unlike in the related art, the complicated preparation process for inducing cancer specificity is not required. Under normal circumstances, lymphocytes can be harvested easily by separating them from peripheral blood. It is desirable to collect the peripheral blood from a vein, and while approximately 0.01 ml ∼ 100 ml of peripheral blood should normally be collected, no specific restrictions are imposed with regard to the quantity. However, in order to ensure that the blood is collected through a simple procedure and the lymphocytes are separated with ease without placing too large a physical onus on the donor, the quantity of the peripheral blood that is collected should be within a range of approximately 5 ml ∼ 50 ml and, more desirably, within a range of 10 ml ∼ 20 ml.

It is to be noted that heparin or citric acid may be added into the collected blood so as to prevent coagulation of the blood. In addition, the lymphocytes may be separated from the collected blood through a lymphocyte separation method in the known art such as the discontinuous density gradient centrifugation executed by sucrose or a commercially available lymphocyte separating agent.

### (Propagation / activation of the lymphocytes)

Next, the cells thus obtained are propagated in a culture by mainly using anti-CD 3 antibodies according to the present invention, and it is desirable to include interleukin 2 in the culture medium solution so as to further improve the propagation efficiency. Accordingly, the lymphocytes are propagated and activated in a culture through combined use of interleukin 2 and anti-CD 3 antibodies in the embodiment.

More specifically, the incubation may be started by suspending the lymphocytes in the culture medium solution containing the interleukin 2 and then placing this culture medium solution in an incubator coated with anti-CD 3 antibodies. In addition, various types of mitogen growth factors and activating factors may be used when propagating and activating the cells as necessary.

No specific restrictions are imposed with regard to the type of anti-CD 3 antibodies used in this process, as long as the propagation / activation of the lymphocytes can be promoted by using it. While the anti-CD 3 antibodies used to stimulate the lymphocytes may be produced in an animal or cells by using refined CD 3 molecules, the commercially available OKT-3 antibody (manufactured by Ortho-Pharmaceutical) achieving outstanding stability and cost performance may be used instead.

In addition, it is desirable to use solid-phase anti-CD3 antibodies in order to achieve better lymphocyte propagation efficiency and better operability. The antibodies may be in solid phase in an incubation container constituted of glass, polyurethane, polyolefine or polystyrene. A commercially-available sterilized cell incubating flask constituted of plastic or the like, which is readily available may be used for these purposes and, in such a case, the size of the flask can be selected as appropriate.

Furthermore, the antibodies can be induced into solid phase by adding a diluted solution of anti-CD3 antibodies into the container used for the purpose of processing the antibodies into solid phase and then leaving the container in a stationary state for 2 ∼ 24 hours with the temperature set to 4 ∼ 37°C. It is desirable that when processing anti-CD3 antibodies into solid phase, anti-CD3 antibodies be diluted to a concentration of 1 ∼ 30µg / ml in a physiological buffer solution such as sterilized Dulbecco's phosphate buffer solution. After the solid phase is achieved, anti-CD3 antibodies may be stored in a cold room or in a refrigerator (4 °C) until it is used. In such a case, the liquid can be removed at the time of use and the anti-CD3 antibodies can be readied for use by washing them with a physiological buffer solution such as Dulbecco's phosphate buffer solution at room temperature.

In addition, the interleukin 2 used in the process is a commercially available product and it should be dissolved so as to achieve a 1 ∼ 2000U/ml concentration in the culture medium solution. The interleukin 2 can be dissolved and used in any medium solution widely used for cell incubation, such as water, a physiological saline solution, a Dulbecco's phosphate buffer solution, RPMI-1640, DMEM, IMDM, and AIM-V. Once the interleukin 2 is dissolved, the solution should be refrigerated for storage so as to ensure that its activity is not reduced.

No specific restrictions are imposed with regard the type of culture medium solution used for this purpose as long as it is suited for the incubation of lymphocytes, and an organism-originating culture solution such as a serum or a synthetic medium achieved by adding amino acids, vitamins, a nucleic acid base and the like into a balanced saline, for instance, may be used. Desirable examples of the culture medium solution include RPMI-1640, AIM-V, DMEM and IMDM, and among these, RPMI-1640 is particularly desirable. Also, it is desirable to use as a culture medium a normal human serum to ensure an outstanding propagating effect. It is to be noted that these culture media are commercially available.

The incubation can be achieved by adopting a standard method of cell incubation such as incubation carried out within a CO2 incubator. When using a CO2 incubator, the CO2 concentration should be maintained within a range of 1 ∼ 10% and more desirably, at approximately 5% and the temperature should be maintained within a range of 30 - 40°C and, more desirably at approximately 37 °C.

### (PDT method)

The PDT is a photodynamic therapy method through which cancerous cells are necrotized through the cytocidal property of the active oxygen generated in the tissue by first intravenously injecting a carcinophilic photosensitive drug, irradiating laser light when the difference between the drug concentration in the cancerous tissue and the drug concentration in the normal tissue reaches a maximum level after 48 - 72 hours and thus exciting the drug having been taken into the cancer.

The laser used in the PDT may be, for instance, a conventional laser generating apparatus such as the excimer dilaser, manufactured by Hamamatsu Photonics K. K., or any other laser generating apparatus that can be used in PDT treatment, such as an argon dilaser, can be utilized.

Since these laser generating apparatuses used in PDT treatment have very small outputs of approximately 1/100 that of a laser scalpel and the carcinophilic drug used in PDT treatment achieves a great concentration at the cancerous tissue, the damage to the normal cells can be minimized and the cancerous nidus can be effectively treated as a sole target.

In addition, the photosensitive substance administered in advance when treating a patient through photodynamic therapy by using laser or the like must demonstrate a specific affinity with the tumor and such a substance can be selected from a wide range of available photosensitive substances that have been used in PDT applications including porfirmer sodium (PHE; commercial name "Photofrin") manufactured by Nippon Wyeth Lederle.

### (Administration of the activated lymphocytes)

It is desirable to administer the activated lymphocytes within 6 months prior to or following the PDT treatment so as to maximize the synergy achieved by combining the activated lymphocyte administration with PDT treatment. In addition, while the activated lymphocytes may be administered only once before the PDT treatment, on the day of PDT treatment or after the PDT treatment, they should be administered in 1 through 10 sessions to strike the right balance between convenience and effect. Under normal circumstances, the activated lymphocytes are administered in a single session or over several sessions, although the effect can be further enhanced by administering them over a greater number of times.

While the activated lymphocytes are administered over five sessions in the embodiment, the present invention is not limited to this example. Also, desired effects can be obtained by administering the activated lymphocytes only prior to the PDT treatment, only on the day of PDT treatment or only after the PDT treatment. However, it is even more desirable to administer them over a plurality of sessions during a six-month period prior to or following the PDT treatment or over a period of time starting and ending respectively before and after the PDT treatment.

In addition, instead of treating the patient only once in the combination of PDT and the activated lymphocyte administration, the patient can be treated with a combination of PDT and the activated lymphocyte administration over a plurality of times. Alternatively, instead of subsequently treating the patient with the combination of PDT and the activated lymphocyte administration, a subsequent treatment may be constituted of a PDT treatment alone or an activated lymphocyte administration alone. Furthermore, if the patient is repeatedly treated with PDT and the activated lymphocytes at different times, one therapy should be implemented within 6 months after the other therapy is implemented so as to assure the desired effect.

### (Cell processing · Assignment)

The present invention may be adopted in a cell processing assignment system in which the harvested lymphocytes are processed or stored at a client's request and then the processed lymphocytes are provided to the client. Namely, the present invention provides activated lymphocytes to be used in conjunction with PDT treatment, obtained at a processing unit by propagating or activating cells contained in collected blood, bodily fluids or tissue provided by a client unit and preparing them as activated lymphocytes to be used in conjunction with PDT, which are then stored or supplied to the client unit, and a cell processing assignment system through which the cells are processed as described above.

It is to be noted that the "client unit" as referred to in this context may be a medical doctor, a dentist, any of various types of medical institutes or a client requesting cells to be processed such as the patient himself or the patient's family, under normal circumstances. In addition, the "processing unit" that processes cells contained in the collected blood, bodily fluid or tissue provided by the client unit by propagating or activating the cells may be a contractor that provides a service of propagating or activating the cells harvested from the blood, the bodily fluid or the tissue provided as described above. Furthermore, a "storage unit" that prepares the processed lymphocytes as frozen cells for storage may be a contractor that provides a service of storing cells in a freezer.

While the client unit, the processing unit and the storage unit may be separate units operating independently of one another, their functions may overlap as well. Accordingly, cells that have been propagated or activated in vitro at the cell processing unit may be stored in a freezer at the processing unit or they may be stored in the freezer at the cell storage unit or the client unit, i.e., the client requesting the cell processing. If the cells are frozen at the cell processing unit, the frozen cells may be stored at the cell processing unit or they may stored at the cell storage unit or the client unit.

Thus, the cells stored in the freezer at the cell processing unit or the cell storage unit can be provided to the client unit (the client requesting the cell storage) in a frozen state, a thawed state or a restored state. In addition, the activated lymphocytes preparation may be suspended in an appropriate solution to facilitate the delivery to the client such as a medical institute via an existing means of transportation such as a courier service.

It is to be noted that a speedy service can be provided via a simple cell processing assignment system through which activated lymphocytes for cancer recurrence prevention are delivered to the client by enabling the client unit to place its order through electronic communication such as e-mail or an Internet homepage.

The activated lymphocytes supplied from the processing unit or the storage unit are administered mainly as an enhancer in the PDT treatment on a patient with stomach cancer. However, the activated lymphocytes according to the present invention may be administered to patients with cancers other than stomach cancer and they may be used as an enhancer in PDT treatment of patients with cancer of the lung, liver, colon, rectum, kidney, spleen, gallbladder, ovary, uterus, testes, prostate, leukemia, sarcoma and brain tumor.

### (Embodiment)

### «1» Separation of lymphocytes

45 ml of peripheral blood was collected from a vein of a patient with stomach cancer whose cancerous tissue has not been reduced in spite of a PDT by adding heparin, to be used directly without preparing it so as to induce cancer specificity. After the peripheral blood was collected, the needle was disengaged from the injection syringe into which the blood had been collected in an aseptic state while ensuring that the connection area was not touched within a clean bench (S-1100 manufactured by Showa Kagaku Co. Ltd.) and a 19G × 1 1/2 needle (available from Nipro Co. Ltd.) was attached to the syringe as a replacement.

15 ml of a washing medium (RPMI 1640+6) (500 ml, manufacturer: Nikken Bio-medical Research Center, GM1106) had been poured into each of two 50 ml centrifugation tubes (manufacturer: Iwaki Glass Co. Ltd., 2341-050), and the blood collected as described above was slowly poured into the two centrifugation tubes so that exactly equal quantities was poured into the two tubes. After completely closing the lids of the centrifugation tubes, they were mixed gently through inversion two or three times.

3 ml of Lymphocepar 1 (100 ml, manufacturer: Immunobiological Bio-research Center Co. Ltd., 23010) was placed into each of six 15 ml centrifugation tubes with a 10 ml pipette (imported by Corning Costar Japan; 4105 ), and then, 10 ml of the blood having been diluted with the medium was slowly stratified over the Lymphocepar 1 so as not to disturb the surface in each centrifugation tube.

The centrifugation tubes were then centrifuged for 15 minutes in a centrifuge at 1800 rpm while maintaining a centrifuging temperature of 20°C in a brake-off state (the centrifuge used in this process was H-700R, manufactured by Kokusan Co. Ltd.). When the centrifugation was completed, the contents of each centrifugation tube were slowly suctioned down to approximately 1 cm above the lymphocyte layer with an aspirator so as not to suction off the lymphocyte cells, while maintaining aseptic conditions. Then, using a 5 ml Pipetman, the lymphocyte cell layer was drawn off without suctioning off the blood clot layer, and the lymphocyte cell layer thus extracted was collected into a 50 ml centrifugation tube into which 25 ml of the washing medium (RPMI 1640+6) had been placed in advance.

After the contents in this centrifugation tube were mixed gently by inversion 2 ∼ 3 times with the lid closed, the centrifugation tube was placed in the centrifuge again to undergo centrifugation for 10 minutes at 1800 rpm with the centrifuging temperature set to 20°C. After the centrifugation, the supernatant fluid was discarded and the cell sediment was thoroughly loosened and stirred.

Then, the cells were mixed gently by inversion in 50 ml of a culture medium achieved by infusing 1 ml of 3500U/ml IL-2 (manufacturer: Cetus Corporation) and 5 ml of human blood serum in 44 ml of a medium (RPMI 1640 + 7; manufacturer; Immunological Bio-research Center Co. Ltd.) and thus, a cell suspension was prepared.

10µl of the cell suspension was taken into a tube (importer/vendor: Assist Co. Ltd., 72.690) and then the suspension was blended with 40µl of Türk solution (manufactured by Mutoh Chemical Co. Ltd.). 10µl of the mixture was placed on a hemocytometer (manufacturer: Elmer Inc., 9731) and the number of cells was measured under a microscope (211320 manufactured by Olympus Optical Industry). The total cell count was 8.5 × 10⁷.

### «2» Preparation of OKT3-coated flask

10 ml of OKT3 solution (importer/vendor: Jansen Kyowa, Co. Ltd., manufacturer: Orthopharmaceutical: OKT3 injection) having been adjusted to achieve a 5µg/ml concentration with PBS (-) was placed into an incubation flask having a base area of 225cm² (MS - 2080R, manufactured by Sumitomo Bakelite) by ensuring the bottom surface of the flask was evenly covered with the solution.

The following day, the OKT3 solution in the flask was suctioned off with an aspirator, and then, 50 ml of PBS (-) was poured into the flask. After the flask was agitated thoroughly with its lid closed, the lid was opened and the liquid was discarded. Next, 50 ml of PBS (-) was poured into the flask while sustaining an aseptic state, then the flask was thoroughly agitated with the lid closed. The lid was then opened and the liquid was discarded. Any moisture remaining inside the flask or on the lid was suctioned off thoroughly with the aspirator and thus, an OKT3-coated flask was prepared.

### «3» Activating incubation of lymphocytes

50 ml of the cell suspension prepared as described in «1» was poured into each OKT3-coated flask prepared as described in «2» and then the suspension was incubated in the flask at 37°C in an environment in which carbon dioxide gas was present at a concentration of 5%. Three days later, 50 ml of the culture medium was added and the incubation was carried on at 37°C in the environment in which the carbon dioxide gas was present at a concentration of 5%. Then, four days later, 150 ml of the culture medium was added and the incubation was carried on at 37°C in the environment in which the carbon dioxide gas was present at a concentration of 5%.

The incubation was allowed to last for another two days at 37°C in the environment in which the carbon dioxide gas was present at a concentration of 5%. As a result, 2.4 × 10⁸ activated lymphocytes were obtained. Of these, 1.2 × 10⁸ cells were suspended in a freeze storage solution and were stored in three separate tube (4.0 × 10⁷ cells / tube) under liquid nitrogen. In addition, the remaining 1.2 × 10⁸ cells were propagated through culture as described below.

### «4» Propagating incubation of lymphocytes (first propagation)

The 1.2 × 10⁸ lymphocytes prepared through «3» above were transferred into a gas permeable incubation bag containing 750 ml of the LL-7 medium (Nikken Bio-medical Research Center) or the Medium 930 (Kojin Bio Co. Ltd.) and then the lymphocytes thus transferred were incubated inside a carbon dioxide gas incubator (CDP-300A; Hirasawa Co. Ltd.) at 37°C within a 5% carbon dioxide gas atmosphere.

Three days later, the gas permeable incubation bag containing the cells and another gas permeable incubation bag containing a new medium were joined by using an aseptic conjugation device (manufacturer: Terumo) the media inside the two gas permeable incubation bags were thoroughly mixed and the medium mixture was divided into two portions. Then, the connection between the bags was cut and after the areas of the junction were aseptically sealed, the cells were continuously incubated at 37°C in a 5% carbon dioxide gas atmosphere.

### «5» Preparing the lymphocytes for administration (first preparation)

The medium containing the cells in one of the two gas permeable bags prepared as described in «4» above was transferred into a centrifugation tube (manufactured by Corning) with a capacity of 250 ml and the cells were separated through centrifugation. Then, the culture solution was eliminated through decantation, a physiological saline solution containing human albumin at a concentration of 0.1 % was added to the cell pellets so as to wash the cells through centrifugation, thereby preparing cell pellets.

Next, 200 ml of physiological saline solution containing human albumin at 1% concentration was added to the cell pellets to suspend the cells and, after the suspension was filtered through a 100µm stainless steel mesh, the preparation was packed into a transfusion bag and was readied for administration. It is to be noted that the number of cells transferred into the transfusion bag was 2.4 × 10⁹.

### «6» Propagating incubation of lymphocytes (second propagation)

Four days later, the other gas permeable incubation bag containing the cells, which was prepared as described in «4» earlier and another gas permeable incubation bag containing a new medium were joined by using an aseptic conjugation device (manufacturer: Terumo), the media inside the two gas permeable incubation bags were thoroughly mixed and the medium mixture was divided into two portions. Then, the connection between the bags was cut and after the areas of the junction were aseptically sealed, the cells were incubated at 37°C in a 5% carbon dioxide gas atmosphere.

### «7» Preparing the lymphocytes for administration (second preparation)

The second preparation of the lymphocytes to be administered was performed in a manner similar to that described in «5» except that the two bags prepared as described in «6» above were used. The final cell count in the transfusion bag was 4.0 × 10⁹.

### «8» Readying cells in freeze storage for use

The frozen cells prepared as described in «3» were thawed at 37°C and then were washed three times with a culture solution. These cells were prepared in a method similar to that described in «3», «4», «5», «6» and «7», thereby obtaining a lymphocyte preparation for administration. Through this process, 3.4 × 10⁹, 5.4 × 10⁹ and 3.5 × 10⁹ activated lymphocytes were prepared.

### «9» PDT treatment

Two days before the laser irradiation on the cancer patient from whom the blood had been collected in «1» described earlier, Photofrin (manufactured by Nippon Wyeth Lederle ) was intravenously injected at a rate of 2mg/kg. Photofrin has characteristics whereby it is taken into cancerous tissue at a rate approximately 10 times higher than the rate at which it is taken into normal tissue, is not eliminated readily from the cancerous tissue and remains in the cancerous tissue at a high concentration. On the day of the laser irradiation, A PDT fiber (manufactured by Hamamatsu Photonics K. K. ) was inserted through the oral cavity, the cancerous lesion was visually verified through imaging and the patient was irradiated with excimer dilaser (manufactured by Hamamatsu Photonics K. K.) five times at 60J/cm². The laser irradiation causes the Photofrin remaining in the cancerous tissue to react and become excited to impart energy with which active oxygen achieving a cytocidal property was generated within the cancerous tissue.

### «10» Administration of lymphocyte preparation

The lymphocyte preparation was administered to the cancer patient to undergo the laser irradiation or having undergone the laser as described in «9» above in the following manner. Namely, the lymphocyte preparations for administration prepared as described in «5», «7» and «8» were intravenously injected for a total of five times, i.e., two weeks prior to the laser irradiation (the number of lymphocytes administered; 2.4 × 10⁹), one week prior to the laser irradiation (the number of lymphocytes administered; 4.0 × 10⁹), on the day of the laser irradiation (the number of lymphocytes administered; 3.4 × 10⁹), one week after the laser irradiation (the number of lymphocytes administered; 5.4 × 10⁹) and three weeks after the laser irradiation (the number of lymphocytes administered; 3.5 × 10⁹).

### «11» Assessment of the effect

In the assessment of effect of the combined treatments which was conducted through ultrasound microscopy and CT scanning twelve days after the laser irradiation, it was observed that the tumor previously swollen to form a range of 4cm at the intra-gastric cavity had been flattened. This clearly demonstrated that even a tumor that could not been effectively treated with a PDT alone could be reduced by combining an activated lymphocyte administration with PDT.

It is to be noted that the activated lymphocytes prepared in «3» as described above may be stored in a freezer as in a specific example explained below. Namely, the activated lymphocytes obtained in «3» are separated through centrifugation, the culture medium is removed through decantation, thereby obtaining cell pellets, 18 ml of a cell preserving solution (prepared by mixing 5 ml of human blood serum, 5 ml of dimethyl sulfoxide (manufacturer: Nakaraitesk Co. Ltd., it may be hereafter referred to as "DMSO") and 40 ml of a medium (RPMI 1640 + 7)) is added to the cell pellets, the cell pellets and the preserving solution are mixed thoroughly, and 3 ml of the mixture is poured into each of five 5ml cell preserving tubes (importer/vendor Corning Costar Japan). These tubes are then placed in liquid nitrogen storage or in an ultra low-temperature freezer and are preserved at low temperature.

In addition, the frozen cells should be thawed and restored for use by taking the frozen cells out of the freeze storage and warming them with a 37°C heat block (manufacturer: TIETECH Inc.; TAL-IG) for 4 minutes. Approximately 3 ml of the cell preserving solution containing the thawed cells is transferred into a 15 ml centrifugation tube under aseptic conditions, the cells are suspended by adding 10 ml of a culture solution or a physiological saline solution and then the cells are separated through centrifugation (executed at 1000 rpm at 20°C over 5 minutes). Afterwards, the supernatant fluid is discarded through decantation, then the cells are suspended by adding 10 ml of the culture solution or the physiological saline solution.

The suspended lymphocytes are further centrifuged (1000 rpm, 20°C, 5 minutes), the supernatant liquid is discarded through decantation, the cells are suspended by adding 10 ml of the culture solution or the physiological saline solution, then the lymphocytes are centrifuged again (1000 rpm, 20°C, 5 minutes) and the supernatant liquid is discarded through decantation so as to allow the cells to be reused for activation propagation, or so as to allow the lymphocytes to be directly used as a preparation for administration by adding 10 ml of physiological saline solution containing human blood serum albumin at a concentration rate of 1% through 5%.

It is to be noted that while an explanation is given above in reference to the embodiment on an example in which patient-originating lymphocytes, i.e., peripheral blood collected from a vein of the stomach cancer patient whose tumor had not been reduced through PDT, were used, donor-originating lymphocytes harvested from a donor achieving the minimum HLA (human leucocyte antigen) match to ensure that GVHD (graft versus host disease) immune deficiency would not be induced were used to obtain a lymphocyte group having been propagated and activated by using anti-CD 3 antibodies in another embodiment, and the treatment administered by using the donor-originating lymphocytes proved to be even more effective in destroying and suppressing cancer such as a tumor compared to the treatment administered by using patient-originating lymphocytes.

### INDUSTRIAL APPLICABILITY

As explained in detail above, according to the present invention, activated lymphocytes are administered in combination with a PDT tumor treatment, and the synergy of the two types of treatments allows the use of activated lymphocytes not necessarily achieving cancer specificity. Thus, the complicated preparation process required to induce cancer specificity no longer needs to be executed and the lymphocytes needed for the therapy can be obtained with greater ease to achieve an outstanding effect in reducing and suppressing various types of tumors such as cancer and in particular stomach cancer. It is of particular interest that even a tumor that cannot be treated effectively with PDT alone can be treated to achieve a highly desirable result.

In addition, lymphocytes originating from a donor achieving the minimum HLA match so as to ensure that GVHD immune deficiency will not be induced, which are then used to obtain a lymphocyte group propagated and activated by using anti-CD 3 antibodies, are even more effective in destroying and suppressing cancers such as tumors compared to patient-originating lymphocytes.

Furthermore, the present invention may be adopted in cancer treatments for animals including house pets such as dogs and cats and livestock such as cattle, pigs, sheep and horses as long as the animal can withstand PDT procedures, as well as in treatment of human cancer. Since the activated lymphocytes used in the present invention can be obtained by propagating and activating lymphocytes with anti-CD 3 antibodies or interleukin 2 and the antineoplastic preparation constituted of the propagated lymphocytes can be preserved in a freezer, a system of assigned propagation processing through which activated lymphocytes to be used in combination with PDT are supplied can be provided by adopting the present invention as well.

Namely, a system of assigned propagation processing through which cells contained in collected blood · bodily fluid or tissue provided by a client unit are first propagated or activated at a processing unit without having to perform a complicated preparation procedure in order to induce cancer specificity, activated lymphocytes to be used in combination with PDT are thus prepared and the activated lymphocytes are then either preserved or supplied to the client unit is achieved. By establishing such a system in a variety of clinical applications, are great improvement can be achieved in success rates in treating difficult tumors.

Moreover, the activated lymphocytes can be stored in a freezer, the frozen lymphocytes can be thawed and restored as necessary and the liquefied lymphocytes or the cells having just underdone the propagation / activation process can be directly used as a preparation to be used in combination with PDT. In addition, the present invention is not limited to the treatment of PDT procedure-eligible cancers, and it may be adopted to treat all types of cancers and tumors through therapies other than PDT that can be combined with the administration of activated lymphocytes.

## Claims

1. A method of tumor treatment **characterized in that**:
activated lymphocytes are administered in combination with a PDT procedure.

2. A method of tumor treatment according to claim 1 **characterized in that**:
said PDT procedure is repeatedly executed over a plurality of sessions.

3. A method of tumor treatment according to claim 1 or claim 2, **characterized in that**:
said activated lymphocytes are administered within 6 months prior to the PDT procedure.

4. A method of tumor treatment according to claim 1 or claim 2, **characterized in that**:
said activated lymphocytes are administered within 6 months after the PDT procedure.

5. A method of tumor treatment according to claim 1 or claim 2, **characterized in that**:
said activated lymphocytes are administered as a concurrent treatment provided within 6 months prior to and also within 6 months after the PDT procedure.

6. A method of tumor treatment according to any of claims 1 through 5, **characterized in that**:
said activated lymphocytes are obtained by propagating or activating lymphocytes with anti-CD 3 antibodies or interleukin 2.

7. A method of tumor treatment according to any of claims 1 through 6, **characterized in that**:
said activated lymphocytes administered in combination with said PDT procedure do not achieve cancer specificity.

8. A preparation to be administered to treat a tumor in combination with a PDT procedure, **characterized in that**:
said preparation contains activated lymphocytes not achieving cancer specificity.

9. A system of assigned propagation processing for propagating and processing lymphocytes to obtain activated lymphocytes to be used in combination with a PDT procedure, **characterized in that**:
said activated lymphocytes to be used in combination with said PDT procedure are prepared at a processing unit by propagating or activating cells contained in collected blood, bodily fluid or tissue provided by a client unit and said activated lymphocytes are stored or supplied to said client unit.

10. A system of assigned propagation processing for propagating and processing lymphocytes to obtain activated lymphocytes to be used in combination with a PDT procedure according to claim 9, **characterized in that**:
said activated lymphocytes prepared to be used in combination with said PDT procedure do not achieve cancer specificity.
